# EUROPEAN PATENT APPLICATION

(11) **EP 1 443 037 A1**
(43) Date of publication of application: **04.08.2004**
(21) Application number: 04076314.6
(22) Date of filing: 23.01.2001
(51) Int. Cl.: C07C 231/10, C07C 233/03, B01J 27/00

(54) **Process for the preparation of substituted formamides**

(30) Priority: 24.01.2000 US 177794 P
(62) Divisional of application: 01942619.6
(71) Applicant: E.I. DUPONT DE NEMOURS AND COMPANY, Wilmington, Delaware 19898 (US)
(72) Inventor: Coulson, Dale Robert, Wilmington, DE 19810 (US); Kourtakis, Kostantinos Dino, Swedesboro, NJ 08085 (US)
(74) Representative: Cockerton, Bruce Roger

(57) **Abstract**

The Present invention provides a process for the preparation of a substituted formamide, said processing comprising:
(i) contacting oxygen in the vapor phase with a compound of the formula CH₃NR₂, wherein R is selected from the group consisting of C₁ to C₈ alkyl, phenyl, naphthyl and C₁ to C₄ alkyl substituted phenyl or mixtures thereof; in the presence of a catalyst, said catalyst selected from the group consisting of:
   (a) xerogels or aerogels of the formula AₓAu_{y}M_{1-(x+y)}, wherein A is selected from the group consisting of Cu, Cr, Ru, Co and combinations thereof, M is selected from the group consisting of Al, Mg, Nb, Si, Ti, Zr and combinations thereof, x is from 0.001 to 0.5 and y is from 0 to 0.2;
   (b) heteropolyacids selected from the group consisting of H₄PMo₁₁VO₄₀, H₅PMo₁₀V₂O₄₀, H₆PMo₉V₃O₄₀ and H₇P₂Mo₁₇VO₆₂;
   (c) metal-substituted heteropolyacids selected from the group consisting of H_{4+(6-z)}PMo₁₀VEO₄₀, H_{5+(6-z)}PMo₉V₂EO₄₀, H_{6+(6-z)}PMo₈V₃EO₄₀ and H_{7+(6-z)}P₂Mo₁₆VEO₆₂, wherein E is an element selected from the group consisting of Cu, Cr, Zn and Co and z is the valence of E;
   (d) heteropolyacid salts selected from the group consisting of Q_{q}H₄PMo₁₁VO₄₀, Q_{q}H₅PMo₁₀V₂O₄₀, Q_{q}H₆PMo₉V₃O₄₀, Q_{q}H₇P₂Mo₁₇VO₆₂, Q_{q}H_{4+(6-z)}PMo₁₀VEO₄₀, Q_{q}H_{5+(6-z)}PMo₉V₂EO₄₀, Q_{q}H_{6+(6-z)}PMo₈V₃EO₄₀ and Q_{q}H_{7+(6-z)}P₂Mo₁₆VEO₆₂ wherein Q is selected from the group consisting of Ag and Cs and q is from 0 to the number of hydrogen atoms multiplied by 0 5 when Q is Ag, and q is from 0 to the number of hydrogen atoms multiplied by 1 when Q is Cs; and
   (e) metal oxides of the formula (BiₜCoᵤNiᵥ)_{w}Mo_{1-w}Oₐ, wherein t, u, and v each are independently from 0 to 1 and t+u+v = 1, w is from 0.01 to 0.75 and a is from 1.125 to 4.875; and
(ii) recovering said substituted formamide.

## Description

### FIELD OF THE INVENTION

The present invention relates to a process wherein a side chain of a tertiary amine is oxidized in the vapor phase in the presence of a catalyst to produce a substituted formamide.

### BACKGROUND

The preparation of substituted formamides from tertiary amines by catalytic oxidation in the liquid phase has been disclosed in U.S. Patent No. 4,543,424 and references cited therein.

U.S. Patent No. 3,483,210 discloses the oxidation of the methyl group of a tertiary N-methylamine to a formyl group using oxygen at atmospheric pressure with the amine in the liquid phase in the presence of a heterogeneous catalyst selected from the platinum group transition metals.

Methylamines are prepared by the vapor phase reaction of methanol and ammonia over an acidic catalyst, such as silica-alumina. The typical mole ratio of amines produced using this catalyst is CH₃NH₂:(CH₃)₂NH:(CH₃)₃N = 17:21:62, the equilibrium value. However, the market demand is greatest for dimethylamine. One of the major uses for this amine is for the preparation of solvents such as dimethylformamide. Typically, trimethylamine is reacted with additional methanol to produce more of the commercially desirable dimethylamine. There is a need for efficient procedures for the utilization of trimethylamine for the preparation of additional dimethylamine or its derivatives such as dimethylformamide.

### SUMMARY OF THE INVENTION

The present invention discloses a process for preparation of a substituted formamide, said process comprising: (i) contacting oxygen in the vapor phase with a compound of the formula CH₃NR₂, wherein R is selected from the group consisting of C₁ to C₈ alkyl, phenyl, naphthyl and C₁ to C₄ alkyl substituted phenyl or mixtures thereof; in the presence of a catalyst selected from the group consisting of: (a) xerogels or aerogels of the formula AₓAu_{y}M_{1-(x+y)}, wherein A is selected from the group consisting of Cu, Cr, Ru, Co and combinations thereof, M is selected from the group consisting of Al, Mg, Nb, Si, Ti, Zr and combinations thereof, x is from 0.001 to 0.5 and y is from 0 to 0.2; (b) heteropolyacids selected from the group consisting of H₄PMo₁₁VO₄₀, H₅PMo₁₀V₂O₄₀, H₆PMo₉V₃O₄₀ and H₇P₂Mo₁₇VO₆₂; (c) metal-substituted heteropolyacids selected from the group consisting of H_{4+(6-z)}PMo₁₀VEO₄₀, H_{5+(6-z)}PMo₉V₂EO₄₀, H_{6+(6-z)}PMo₈V₃EO₄₀ and H_{7+(6-z)}P₂Mo₁₆VEO₆₂, wherein E is an element selected from the group consisting of Cu, Cr, Zn and Co and z is the valence of E; (d) heteropolyacid salts selected from the group consisting of Q_{q}H₄PMo₁₁VO₄₀, Q_{q}H₅PMo₁₀V₂O₄₀, Q_{q}H₆PMo₉V₃O₄₀, Q_{q}H₇P₂Mo₁₇VO₆₂, Q_{q}H_{4+(6-z)}PMo₁₀VEO₄₀, Q_{q}H_{5+(6-z)}PMo₉V₂EO₄₀, Q_{q}H_{6+(6-z)}PMo₈V₃EO₄₀ and Q_{q}H_{7+(6-z)}P₂Mo₁₆VEO₆₂ wherein Q is selected from the group consisting of Ag and Cs and q is from 0 to the number of hydrogen atoms multiplied by 0.5 when Q is Ag, and q is from 0 to the number of hydrogen atoms multiplied by 1 when Q is Cs; (e) a xerogel or aerogel composite of the formula Ruᵣ(HPA)ₛM₁₋₍ᵣ₊ₛ₎, wherein r is from 0.001 to 0.1, s is from 0.001 to 0.05, HPA is selected from the group consisting of heteropolyacids and heteropolyacid salts of catalysts (b) and (c) and M is selected from the group consisting of Al, Mg, Nb, Si, Ti, Zr; and (f) metal oxides of the formula (BiₜCoᵤNiᵥ)_{w}Mo _{l-w}Oₐ, wherein t, u, and v each are independently from 0 to I and t+u+v = 1, w is from 0.01 to 0.75 and a is from 1.125 to 4.875; and (ii) recovering an N-substituted formamide of the formula HC(O)NR₂.

Also disclosed is a composition of matter, comprising: xerogels or aerogels of the formula AₓAu_{y}M_{1-(x+y)}, wherein A is selected from the group consisting of Cu, Cr, Ru, Co and combinations thereof, M is selected from the group consisting of Al, Mg, Nb, Si, Ti, Zr and combinations thereof, wherein x is from 0.001 to 0.5 and wherein y is from 0 to 0.2.

A further disclosure is a composition of matter, comprising: a xerogel or aerogel composite of the formula Ruᵣ(HPA)ₛM₁₋₍ᵣ₊ₛ₎, wherein r is from 0.001 to 0.1, s is from 0.001 to 0.05, HPA is selected from the group consisting of heteropolyacids and heteropolyacid salts and M is selected from the group consisting of Al, Mg, Nb, Si, Ti, Zr.

### DETAILED DESCRIPTION

The tertiary amine starting material to be oxidized can be any that conforms to the formula CH₃NR₂ wherein R is selected from the group consisting of C₁ to C₈ alkyl, phenyl, naphthyl and C₁ to C₄ alkyl substituted phenyl or mixtures thereof. The oxidation product will be any formamide corresponding to the formula HC(O)NR₂. The oxidation of trimethylamine to dimethyl formamide is preferred.

The oxygen used in the process can be pure, mixed with an inert gas, or air. The use of air is preferred.

The reaction is typically conducted at temperatures of from about 125°C to about 450°C, preferably from about 150°C to about 275°C. The reaction pressure is typically from about 1 atmosphere (100 kPa) to about 10 (1000 kPa) atmospheres.

The oxidation products are recovered from the product mixtures by conventional techniques such as fractional distillation.

The catalysts described in the present invention can be prepared by a variety of known art methods such as impregnation, xerogel or aerogel formation, formation of polyoxometallates by refluxing and ion exchange, freeze-drying, spray drying, and spray roasting. In addition to catalyst powders, extrudates and pellets, monoliths can be used as supports provided that they have sufficient porosity for reactor use.

The xerogels or aerogels used in this invention comprise a matrix material which is derived from a solution of the matrix component(s) and which incorporates the active catalyst component(s) which is derived from one or more dissolved component(s). A matrix is a skeletal framework of oxides and oxyhydroxides derived from the hydrolysis and condensation of alkoxides with other reagents. The framework typically comprises 30% or more by weight of the total catalyst composition. The matrix material comprises magnesium, silicon, titanium, zirconium or aluminum, oxide/hydroxide xerogels or aerogels or mixtures thereof totaling from 30 mole % to 99.9 mole %, preferably from 65 to 85 mole % of the catalyst composition.

In the present invention one or more metal alkoxides (for example, aluminum isopropoxide) may be used as starting material for preparing the gels. The inorganic metal alkoxides used in this invention may include any alkoxide which contains from 1 to 20 carbon atoms, and preferably 1 to 5 carbon atoms in the alkoxide group, which preferably are soluble in the liquid reaction medium. C₁-C₄ alkoxides such as aluminum isopropoxide, titanium isopropoxide and zirconium isopropoxide are preferred.

Commercially available alkoxides can be used. However, inorganic alkoxides can be prepared by other routes. Some examples include direct reaction of zero valent metals with alcohols in the presence of a catalyst. Many alkoxides can be formed by reaction of metal halides with alcohols. Alkoxy derivatives can be synthesized by the reaction of the alkoxide with alcohol in a ligand interchange reaction. Direct reactions of metal dialkylamides with alcohol also form alkoxide derivatives. Additional examples are disclosed in "Metal Alkoxides" by D. C. Bradley et al., Academic Press, (1978).

The first step in the synthesis of the gels containing alcohol, or alcogels, comprises first of preparing non-aqueous solutions of the alkoxides and other reagents and separate solutions containing protic solvents, such as water. When the alkoxide solutions are mixed with the solutions containing the protic solvents, the alkoxides and polymerize to form a gel.

The solution medium used in the process generally should be a solvent for the inorganic alkoxide or alkoxides utilized and the additional metal reagents and promoters added in the single step synthesis. Solubility of all components in their respective media (aqueous and non-aqueous) is preferred to produce highly dispersed materials. By employing soluble reagents in this manner, mixing and dispersion of the active metals and promoter reagents can be near atomic, in fact mirroring their dispersion in their respective solutions. The precursor gel produced by this process will contain highly dispersed active metals and promoters. High dispersion results in catalyst metal particles in the nanometer size range.

Of note are embodiments where the catalytic metal component of the gel is dissolved in a separate protic solvent (for example, water) and this solution of catalytic metal compound(s) is mixed with the non-aqueous solution of the matrix component(s). Also of note are embodiments where the catalytic metal component is dissolved in the same non-aqueous solution as the matrix component(s), and an aqueous supplement is used.

Typically, the concentration of the amount of solvent used is linked to the alkoxide content. A molar ratio of 26.5:1 ethanol:total alkoxide can be used, although the molar ratio of ethanol:total alkoxide can be from about 5:1 to 53:1, or even greater. If a large excess of alcohol is used, gelation will not generally occur immediately and some solvent evaporation will be needed. At lower solvent concentrations, it is probable that a heavier gel will be formed that has no less pore volume and surface area.

In the present invention, to the alcohol soluble alkoxide and other reagents, water and any aqueous solutions are added in a dropwise fashion to induce hydrolysis and condensation. Depending on the alkoxide system, a discernible gel point can be reached in minutes or hours. The molar ratio of the total water added to total AI, Mg, Nb, Si, Ti and Zr added (including water present in aqueous solutions) varies according to the specific inorganic alkoxide being reacted.

Generally, a molar ratio of water:alkoxide from about of 0.1:1 to 10:1 is used. However, ratios close to 4:1 for zirconium(alkoxide)₄ and titanium(alkoxide)₄ can be used, 2:1 for Mg(alkoxide)₂, 3:1 for Al(alkoxide)₃, 5:1 for Nb(alkoxide)₅, 4:1 for Si(alkoxide). The amount of water utilized in the reaction is that calculated to hydrolyze the inorganic alkoxide in the reaction mixture. A ratio lower than that needed to hydrolyze the alkoxide species will result in a partially hydrolyzed material, which in most cases will reach a gel point at a much slower rate, depending on the aging procedure and the presence of atmospheric moisture.

The addition of acidic or basic reagents to the inorganic alkoxide medium can have an effect on the kinetics of the hydrolysis and condensation reactions, and the microstructure of the oxide/hydroxide matrices derived from the alkoxide precursor that entraps or incorporates the soluble metal and promoter reagents. Generally, a pH within the range of from 1 to 12 can be used. A pH range of from 1 to 6 being preferred.

After reacting to form the alcogels of the present invention, it may be necessary to complete the gelation process with some aging of the gel. This aging can range from one minute to several days. In general, all alcogels are aged at room temperature in air for at least several hours.

Removal of solvent from the alcogels can be accomplished by several methods. Removal by vacuum drying or heating in air results in the formation of a xerogel. An aerogel of the material can typically be formed by charging in a pressurized system such as an autoclave. The solvent-containing gel that is formed in the present invention is placed in an autoclave where it can be contacted with a fluid above its critical temperature and pressure by allowing supercritical fluid to flow through the gel material until the solvent is no longer being extracted by the supercritical fluid. In performing this extraction to produce an aerogel material, various fluids can be utilized at their critical temperature and pressure. Examples of such fluids that are suitable for this process include, but are not limited to, fluorochlorocarbons typified by Freon® fluorochloromethanes (e.g., Freon® 11 (CCl₃F), Freon® 12 (CCl₂F₂) or Freon® 114 (CClF₂CClF₂), ammonia and carbon dioxide. Typically, the extraction fluids are gases at atmospheric conditions so that pore collapse due to the capillary forces at the liquid/solid interface are avoided during drying. In most cases, the resulting material possesses a higher surface area than the non-supercritically dried materials.

The xerogels and aerogels thus produced can be described as precursor salts dispersed in an oxide or oxyhydroxide matrix. A theoretical maximum of hydroxyl content corresponds to the valence of central metal atom. The molar H₂O:alkoxide ratio can also impact the final xerogel stoichiometry so that there will be residual -OR groups in the unaged gel. However, reaction with atmospheric moisture will convert these to the corresponding -OH, and -O groups upon continued polymerization and dehydration. Aging, even under inert conditions, can also effect the condensation of the -OH, eliminating H₂O, through continuation of cross linking and polymerization, i.e., gel formation. In part, the degree of crosslinking and conversion of -OH is indicated in the variable stochiometry for the hydroxyl content indicated in the formula. For example, AlO_{1.5-z}(OH)_{2z}, where z is variable and can range from 1.5 (entirely hydroxyl) to 0 (entirely oxide).

In another embodiment of the present invention one or more inorganic metal colloids may be used as starting material for preparing the gels. These colloids include colloidal alumina sols, colloidal zirconia sols, colloidal titania sols, mixed oxide colloid sols, colloidal silica sols, ternary or higher order oxide sols, and mixtures thereof. Several of the colloidal sols are commercially available. There are also several methods of preparing colloids, as described in "Inorganic Colloid Chemistry", Volumes 1, 2, and 3, J. Wiley and Sons, Inc., 1935. Colloid formation involves either nucleation and growth, or subdivision or dispersion processes. For example, hydrous titanium dioxide sols can be prepared by adding ammonia hydroxide to a solution of a tetravalent titanium salt, followed by peptization (re-dispersion) by dilute alkalis. Zirconium oxide sol can be prepared by dialysis of sodium oxychlorides. The preparation of other sols are described in these references.

Commercially available alkoxides, such as tetraethylorthosilicate and organic titanate esters (sold by the DuPont Company under the tradename "Tyzor®") can be used. Also, inorganic alkoxides can be prepared by various routes. Examples include direct reaction of zero valent metal with alcohols in the presence of a suitable catalyst and the reaction of metal halides with alcohols. Alkoxy derivatives can be synthesized by the reaction of the alkoxide with alcohol in a ligand interchange reaction. Direct reactions of metal dialkylamides with alcohol also form alkoxide derivatives. Additional examples are disclosed in D. C. Bradley et al., Metal Alkoxides (Academic Press, 1978).

In a preferred embodiment of the process of this invention, pre-formed colloidal sols in water, or aquasols, are used. The aquasols are comprised of colloidal particles ranging in size from 2 to 50 nanometers. In general, the smaller primary particle sizes (2 to 5 nm) are preferred. The pre-formed colloids contain from 10 to 35 weight % of colloidal oxides or other materials, depending on the method of stabilization. Generally, after addition of the active (for the partial oxidation reactions, either as a catalyst or promoter) metal components, the final de-stabilized colloids can possess from about 1 to 35 weight % solids, preferably from about 1 to 20 weight %.

The colloidal oxides, or their mixtures, are destabilized during the addition of soluble salts of the primary and promoter cation species by the addition of acids or bases or by solvent removal, both of which alter pH. Once the particles come in close enough contact when destabilized, polymerization and crosslinking reaction between surface functional groups, such as surface hydroxyls, can occur. In one embodiment of this invention the colloids, which are originally stable heterogeneous dispersions of oxides and other species in solvents, are destabilized to produce colloidal gels. Destabilization is induced, in some cases, by the addition of soluble salts, e.g., chlorides or nitrates, which change the pH and the ionic strength of the colloidal suspensions by the addition of acids or bases, or by solvent removal. pH changes generally accompany the addition of soluble salts. In general, this is preferred over solvent removal. Generally, a pH range of from about 0 to about 12 can be used to destabilize the colloids, however, very large extremes in pH can cause flocculation and precipitation. For this reason, a pH range of from about 2 to 8 is preferred, generally.

The medium utilized in this process is typically aqueous, although non-aqueous colloids can also be used. The additional metal or inorganic reagents (i.e., salts of Cr or promoters) used should be soluble in the appropriate aqueous and non-aqueous media.

Removal of solvent from the gels can be accomplished by several methods as described above to prepare either an aerogel or xerogel.

The heteropolyacid (HPA) catalysts (catalysts (b), (c), and (d)) of this invention are typically prepared by an aqueous process. Amounts of the appropriate reagents, which are the amounts calculated to give the desired transition metal substituted vanadium promoted heteropolyacid, are combined in water and heated to elevated temperatures. A convenient temperature is reflux (100°C), and a convenient time period is overnight. However, time and temperature are not critical as long as the time/temperature is sufficient for all of the reagents to go into solution and give a clear, usually highly colored, solution. In some cases the reaction is complete within two hours at reflux, in other cases longer time is required.

Preferably, the reaction is performed in an air atmosphere. Inert atmospheres may also be employed. The process is usually conducted at normal atmospheric pressure, but elevated or reduced pressures may also be employed. Agitation is not required, but is usually provided to facilitate heat transfer.

Commercially available reagents are used for the HPA preparation. The purity of the reagents should be known so that the gross amount required may be calculated. The amount of reagent employed should be within plus or minus 5%, preferably within plus or minus 2%, of the amount indicated by stoichiometry.

The product is isolated by evaporating the reaction mixture to dryness. This may be done by any known method, typically employing a vacuum to speed the process. The isolated product may be used as is, or in the cases where certain equipment is employed for the subsequent use of the HPA's, the product HPA's may be processed or fabricated into various size and shape particles before use. That is, the product may be ground, pelletized, briquetted tabulated, or shaped in other ways as required for use in certain equipment.

Variants of the above described preparative procedure include preparation of an ammonium salt of the desired HPA followed by the thermal removal of ammonia. A second alternative is to generate the desired acid form of the HPA via ion exchange.

A xerogel or aerogel composite of a heteropolyacid and an appropriate sol gel derived material can also be used in the present invention, which is described in the Summary of the Invention section as catalyst (e). These composites can be synthesized by adding the water soluble heteropolyacid to the alkoxide solution during the hydrolysis of the alkoxide or to the aquasol before or during the destabilization of the aquasol to produce the gel material. During these additions, other inorganic reagents can be added (for example, Ru as a cationic species which can serve as a counterion on the heteropolyacid).

Freeze drying procedures can be used for several catalyst compositions, (such as those described in the Summary of the Invention as catalyst (f)), and are useful if the catalyst precursors are soluble in water or other solvent which can be rapidly (less than 1 minute) frozen. Precursor salts are dissolved in an appropriate amount of solvent to form a solution or fine colloid. The solution is then rapidly cooled and frozen by immersion in a suitable medium, such as liquid nitrogen. If the solution is rapidly frozen, the salts and other components will remain intimately mixed and will not segregate to any significant degree. The frozen solid is transferred to a freeze drying chamber. The solution is kept frozen while water vapor is removed by evacuation.

Spray drying procedures involve the use of solutions, colloids or slurries containing catalyst precursors or catalyst compounds described in the Summary of the Invention section above as catalysts (a), (b), (c), (d), (e), and (f). The technique consists of atomization of these liquids (usually, but not exclusively, aqueous) into a spray, and contact between spray and drying medium (usually hot air) resulting in moisture evaporation. The drying of the spray proceeds until the desired moisture content in the dried particles is obtained, and the product is recovered by suitable separation techniques (usually cyclone separation). A detailed description of spray drying methods can be found in Spray Drying Handbook, 4th edition by K Masters (Longman Scientific and Technical, John Wiley and Sons, NY) c. 1985.

Spray roasting also involves the use of solutions or colloids, but generally involves drying and calcination (at higher temperatures) in one process step to produce catalyst powders.

### EXAMPLES

For experiments described in this invention, a two section Virtis freeze drying unit was employed. Refrigerated shelves were used to prevent thaw-out of the frozen solids during evacuation. All percentages in the following Examples are by weight unless otherwise indicated.

### General Procedure for Catalyst Testing

In a typical test a 1/4" (6.4 mm) stainless steel reactor was loaded with a fixed volume (0.625 mL) of catalyst (with silica wool used to hold it in place) and was heated to the initial reaction temperature (175°C) under nitrogen. The gas feed system is configured to deliver a mixture of 1.5% trimethylamine/20% oxygen/78.5% nitrogen to the reactor at a constant flowrate of 20.9 cc/min and one atmosphere pressure. Prior to taking a reactor sample, a sample of the unreacted feed gas is taken to establish the gas composition. Analysis of the gas mixture was by GC. A Hewlett-Packard Model 5890 gas chromatography unit containing two detectors (flame ionization and thermal conductivity) was used to analyze the effluent gases. The columns used included: (1) a combination of a 10' (3.05 m) x 1/8" (3.2 mm) stainless steel 60/80 mesh (0.25/0.18 mm) Molecular Sieve 13X column (used to separate H₂O, N₂ and CO) and a 2' (0.61 m) x 1/8" (3.2 mm) stainless steel 80/100 mesh (0.18/0.15 mm) Haysep R® (a co-polymer of divinylbenzene and N-vinyl-2-pyrollidone) column (used to separate H₂O, CO₂ and butane), and (2) a 20 m x 0.53 mm DB1 (dimethylpolysiloxane) capillary column used to separate the organics. Helium was used as a carrier gas for all columns. Analyses on both column systems were carried out simultaneously using separate samples taken from two 500 microliter sample loops. A valve switching scheme was used to insure that each sample was properly analyzed. The GC was programmed to control the column temperatures in a manner such that a total analysis could be completed in 15.45 minutes.

The response factors for certain compounds were determined using two methods: (1) syringe injection of gases or liquids or (2) sample loop injections of gases.

Following the analysis of feed gases, samples of reacted gases were taken at each of three or four temperatures (175°, 225°, 275° and again at 175°C) and analyzed the same as above. The test was completed by taking a final feed gas sample for analysis. The data was then compiled and calculations carried out to determine the % conversion of trimethylamine and the selectivities to dimethylformamide and monomethylformamide.

Catalyst test results are shown in Tables 1 to 5.

| Legend | |
|---|---|
| T is Temperature | CT is contact time |
| TMA is (CH₃)₃N | DMF is (CH₃)₂NCHO |
| MMF is CH₃NHCHO | |

### EXAMPLE 1

A 0.349 M solution of magnesium methoxide in ethanol (47.33 mL) was added to a 150 mL petri dish with gentle swirling, along with a 0.02 M ethanolic solution of AuCl₃ (21.776 mL). In a second step, an aqueous chromium hydroxide acetate solution (Cr₃(OH)₂(CH₃CO₂)₇, 0.871 mL (0.5 M)) was slowly added. A gel point was realized, and the material was allowed to age at least 24 hours prior to use. After drying at 120°C under vacuum for 5 hours, the material was calcined at 250°C in air for 1 hour. The calcined powder was pelletized at 20,000 psig (138 mPa) and granulated on -40, +60 mesh (-0.42, +0.25 mm) screens prior to use. The nominal metal composition of the catalyst was Au_{0.025}Cr_{0.025}Mg_{0.95}.

### EXAMPLE 2

The same procedure as described in Example 1 was followed, except that the following amounts of reagents were used: 0.05 M solution of aluminum isopropoxide in ethanol (68.19 mL), a 0.02 M ethanolic solution of AuCl₃ (1.740 mL) and an aqueous chromium hydroxide acetate solution (Cr₃(OH)₂(CH₃CO₂)₇, 0.07 mL (0.5 M)). The nominal metal composition of the catalyst was Au_{0.01}Cr_{0.01}Al_{0.98}.

### EXAMPLE 3

The same procedure as described in Example 1 was followed, except that the following amounts of reagents were used: 0.6 vol. % solution of tetraethyl orthosilicate in ethanol (0.317 mL), a 0.02 M ethanolic solution of AuCl₃ (22.436 mL), a 0.349 M solution of magnesium methoxide in ethanol (46.349 mL) and an aqueous chromium hydroxide acetate solution (Cr₃(OH)₂(CH₃CO₂)₇, 0.897 mL (0.5 M)). The nominal metal composition of the catalyst was Au_{0.025}Mg_{0.9025}Cr_{0.015}Si_{0.0475}.

### EXAMPLE 4

The same procedure as described in Example 1 was followed, except that the following amounts of reagents were used: a 0.5 M solution of aluminum isopropoxide in ethanol (69.821 mL) and an aqueous chromium hydroxide acetate solution (Cr₃(OH)₂(CH₃CO₂)₇, 0.179 mL (0.5 M)). The nominal metal composition of the catalyst was Cr_{0.025}Al_{0.975}.

### EXAMPLE 5

The same general procedure as described in Example 1 was followed, except that the reagents and sioichiomeries were adjusted. AuCl₃ (0.9579 g) was dissolved in ethanol (50 mL) and added to aluminum isopropoxide (24.51 g) dissolved in isopropanol (2500 mL). The dissolution was done in an inert atmosphere drybox. An aqueous chromium solution prepared by dissolving of chromium hydroxide acetate (0.6351 g) in (1 ml) water followed by diluting the solution with ethanol (10 mL). The nominal metal composition of the catalyst was Au_{0.025}Al_{0.95}Cr_{0.025}.

### EXAMPLE 6

The same procedure as described in Example 1 was followed, except that the following amounts of reagents were used: 0.05 M solution of aluminum isopropoxide in ethanol (64.842 mL), a 0.02 M ethanolic solution of AuCl₃ (4.49 mL), a 0.349 M solution of magnesium methoxide in ethanol (0.488 mL) and an aqueous chromium hydroxide acetate solution (Cr₃(OH)₂(CH₃CO₂)₇, 0.180 mL (0.5 M)). The nominal metal composition of the catalyst was Au_{0.075}Cr_{0.025}Al_{0.9025}Mg_{0.0475}.

### EXAMPLE 7

The same procedure as described in Example I was followed, except that the following amounts of reagents were used: 0.05 M solution of aluminum isopropoxide in ethanol (64.842 mL), a 0.02 M ethanolic solution of AuCl₃ (4.518 mL), a 0.6 vol. % solution of tetraethylorthosilicate in ethanol (0.064 mL) and an aqueous chromium hydroxide acetate solution (Cr₃(OH)₂(CH₃CO₂)₇, 0.181 mL (0.5 M)). The nominal metal composition of the catalyst was Au_{0.025}Cr_{0.025}Al_{0.9025}Si_{0.0475}.

### EXAMPLE 8

The same procedure as described in Example 1 was followed, except that the following amounts of reagents were used: 0.05 M solution of aluminum isopropoxide in ethanol (51.21 mL), a 0.02 M ethanolic solution of AuCl₃ (10.107 mL), a 0.6 vol. % solution of tetraethylorthosilicate in ethanol (0.952 mL), a 0.349 M solution of magnesium methoxide in ethanol (7.326 mL) and an aqueous chromium hydroxide acetate solution (Cr₃(OH)₂(CH₃CO₂)₇, 0.404 mL (0.5 M)). The nominal metal composition of the catalyst was Au_{0.025}Cr_{0.025}Mg_{0.32}Al_{0.32}Si_{0.32}.

### EXAMPLE 9

The same procedure as described in Example 1 was followed, except that the following amounts of reagents were used: a 0.02 M ethanolic solution of AuCl₃ (49.396 mL), a 0.6 vol. % solution of tetraethylorthosilicate in ethanol (13.25 mL), a 0.349 M solution of magnesium methoxide in ethanol (5.371 mL) and an aqueous chromium hydroxide acetate solution (Cr₃(OH)₂(CH₃CO₂)₇, 1.976 mL (0.5 M)). The nominal metal composition of the catalyst was Au_{.025}Cr_{.025}Si_{.9025}Mg_{.0475}.

### EXAMPLE 10

Water (1000 mL), MoO₃ (122.4 g) and vanadium pentoxide (4.6 g) were added to a 3 liter round bottom flask equipped with an addition funnel, mechanical stirrer and a reflux condenser. The slurry was brought to reflux. Aqueous phosphoric acid (11.5 g) was then slowly added over the course of 20 minutes, until the desired stoichiometry was achieved. After about 3 hours, an orange-red solution formed. Typically, the reflux was continued for 16 hours (overnight). The samples were dried at 120°C for approximately 12 hours in nitrogen. The catalyst composition was H₇P₂Mo₁₇VO₆₂.

### EXAMPLE 11

The same procedure as described in Example 10 was used. Water (1000 mL), MoO₃ (100.2 g), vanadium pentoxide (6.33 g) and CuO (5.5 g) were added to a 3 liter round bottom flask equipped with an addition funnel, mechanical stirrer and a reflux condenser. The slurry was brought to reflux. Aqueous phosphoric acid (8.1 g) was then slowly added over the course of 20 minutes, until the desired stoichiometry was achieved. After about 3 hours, an orange-red solution formed. Typically, the reflux was continued for 16 hours (overnight). The samples were dried at 120°C for approximately 12 hours in nitrogen. The catalyst composition was HₚPMo₁₀CuVO₄₀, where is p 8 and 9.

### EXAMPLE 12

The same procedure described in Example 11 was used with the following reagents: water (1000 mL), MoO₃ (100.2 g), vanadium pentoxide (6.3 g), CuO (1.1 g), Cr(NO₃)₃•9H₂O (27.8 g) and aqueous phosphoric acid (8.0 g). The catalyst composition was Cu_{0.2}HₙPMo₁₀VCrO_{40,} where n 3.6 3.8.

### EXAMPLE 13

The same procedure described in Example 11 was used with the following reagents: water (1000 mL), MoO₃ (100.2 g), vanadium pentoxide (6.3 g), ZnCl₂ (9.5 g) and aqueous phosphoric acid (8.01 g). The catalyst composition was H₈PMo₁₀VZnO₄₀.

### EXAMPLE 14

The procedure disclosed in patent U.S. Patent No. 4,192,951 was used to prepare a H₄PMo₁₁VO₄₀. Sodium phosphate, dibasic (Na₂HPO₄•7H₂O, 48.75 g) was dissolved in refluxing H₂O (100 mL). A second solution containing sodium metavanadate (NaVO₃, 22.17 g) in H₂O (150 mL) was prepared. A third solution containing sodium molybdate (Na₂MoO₄•2H₂O, 411.98 g) in H₂O (500 mL) was prepared. The three solutions were then combined with sulfuric acid, H₂SO₄ (205.4 mL). The combined solution was then added to a 250 mL separatory funnel. The heteropolyacid was separated by adding diethyl ether to make the appropriate heteropoly etherate, which was then removed and dried to make the free acid. The heteropolyacid was dissolved in water. Silver carbonate was added to produce the heteropolyacid of the appropriate stoichiometry. The material was evaporated to dryness prior to use. The catalyst composition was H₂Ag₂PMo₁₁VO₄₀.

### EXAMPLE 15

The same procedure as described in Example 10 was used. To a five liter, three neck round bottom flask (fitted with a heating mantle for refluxing), molybdenum oxide (MoO₃, 440.9 g), vanadium oxide (V₂O₅, 25.3 g) and water (4000 mL) were added. After bringing to reflux, 85 wt % phosphoric acid (32.0 g) was added. After about 16 hours of reflux, a red - brown solution formed. The material was evaporated to dryness to produce the free acid. The heteropolyacid was dissolved in water. Silver carbonate was added to produce the heteropolyacid of the appropriate stoichiometry. The material was evaporated to dryness prior to use. The catalyst composition was H₃AgPMo₁₁VO₄₀.

### EXAMPLE 16

The same procedure described in Example 10 was used with the following reagents: water (1000 mL), MoO₃ (9.0 g), vanadium pentoxide (1.27 g), CuO (0.55 g) and aqueous phosphoric acid (1.6 g). The catalyst composition was HₘPMo₉V₂CuO₄₀, where m 9 10.

### EXAMPLE 17

The same procedure described in Example 10 was used with the following reagents: water (1000 mL), MoO₃ (100.2 g), vanadium pentoxide (6.33 g), cobalt 2,4-pentanedionate (17.9 g) and aqueous phosphoric acid (8.01 g). The catalyst composition was H₈PMo₁₀VCoO₄₀.

### EXAMPLE 18

A heteropolyacid, "H₄PMo₁₁VO₄₀" was prepared in the following manner; to a five liter, three neck round bottom flask (fitted with a heating mantle for refluxing), molybdenum oxide (MoO₃, 440.9 g). vanadium oxide (V₂O₅, 25.3 g) and water (4000 mL) were added. After bringing to reflux, 85 wt % phosphoric acid (32.0 g) was added. After about 16 hours of reflux, a red - brown solution formed. The material was evaporated to dryness to produce the free acid. A portion of this material(4.453 g) was dissolved in water. Cs₂CO₃ (1.63 g) to form the final stoichiometry. The material was dried. The catalyst composition was Cs₄PMo₁₁VO₄.

### EXAMPLE 19

An aerogel composite of the heteropolyacid, H₄PMo₁₁VO₄₀, was prepared. Titanium n-butoxide (183.794 g) was added to a 1.5 liter resin kettle in an inert atmosphere drybox along with ethanol (206.45 mL). A second solution was prepared in a dropping funnel, containing ruthenium trichloride (6.23 g) in water (38.91 mL), glacial acetic acid (4.634 mL), nitric acid (3.618 mL), ethanol (206.45 mL) and, H₄PMo₁₁VO₄₀ (53.437 g), prepared as described in Example 18 were dissolved in water and then added to the alkoxide solution. In this case, the alcohol: metal molar ratio is 26.5:1. The 1.5 liter resin kettle was under a nitrogen purge during this addition, and the solution was gently stirred. A gel material was formed, which was aged for 24 hours at room temperature, pelletized at 20,000 psig (138 mPa) and granulated on -40, +60 mesh (-0.42, +0.25 mm) screens prior to use. The nominal catalyst composition was Ru_{0.05}(H₄PMo₁₁VO₄₀)_{0.05}(TiO₂)_{0.9}.

### EXAMPLE 20

An identical procedure as described in Example 19 was followed, except that the reagents and stoichiometries used were adjusted as indicated below. No heteropolyacid was added in this preparation. In an inert atmosphere drybox (under nitrogen) ethanol (412.5 mL) was used to dissolve zirconium isopropoxide (176.882 g). This was added to a 1.5 liter resin kettle. In a second dropping funnel, a solution containing ethanol (412.5 mL) was used in combination with the other aqueous solutions (prepared by dissolving RuCl₃ (6.223 g) and H₄PMo₁₁VO₄₀ (53.438 g) in H₂O (38.913 mL) containing HNO₃ (3.619 mL) and acetic acid (4.635 mL)) were added, in a dropwise fashion, to the zirconium isopropoxide solution. The H₂O:alkoxide ratio was 4:1. During the addition, the resin kettle was blanketed with nitrogen and the solution was stirred during the addition. The nominal catalyst composition was Ru_{0.05}(H₄PMo₁₁VO₄₀)_{0.05}(ZrO₂)_{0.9}.

### EXAMPLE 21

An identical procedure as described in Example 20 was followed, except that the reagents and stoichiometries used were adjusted, as indicated below. Ethanol (344 mL) was used to dissolve niobium ethoxide (143.195 g). Aqueous solutions were prepared by dissolving RuCl₃ (5.186 g) and H₄PMo₁₁VO₄₀ (44.532 g) in H₂O (40.534 nL) containing HNO₃ (3.77 mL), acetic acid (4.828 mL) and ethanol (344 mL). The water:alkoxide ratio was 5:1. The nominal catalyst composition was Ru_{0.05}(H₄PMo₁₁VO₄₀)_{0.05}(NbO_{2.5})_{0.9}.

For Examples 22 to 28 and 30, l is ≥ 0 and 1.

### EXAMPLE 22

An identical procedure as described in Example 20 was employed, except that the reagents and stoichiometries used were adjusted as indicated below. Ti-n-butoxide (163.373 g) was dissolved in ethanol (371.21 mL). An aqueous chromium hyroxide acetate solution was prepared by dissolving chromium hydroxide acetate (24.1332 g) in H₂O (35.489 mL) and glacial acetic acid (8.24 mL). The H₂O:alkoxide ratio was 4:1. The nominal catalyst composition was Cr_{0.2}(TiO₂₋₁(OH)₂₁)_{0.8}.

### EXAMPLE 23

An identical procedure as described in Example 20 was employed, except that the reagents and stoichiometries used were adjusted as indicated below. Tetraethylorthosilicate (163.373 g) was dissolved in ethanol (371.21 mL). An aqueous chromium hyroxide acetate solution was prepared by dissolving chromium hydroxide acetate (24.1332 g) in H₂O (34.589 mL). The H₂O:alkoxide ratio was 4:1. The nominal catalyst composition was Cr_{0.2}(SiO₂₋₁(OH)₂₁)_{0.8}.

### EXAMPLE 24

An identical procedure as described in Example 20 was employed, except that the reagents and stoichiometries used were adjusted as indicated below. Tetraethylorthosilicate (74.9988 g) was dissolved in ethanol (278.4 mL). An aqueous solution consisting of chromium hydroxide acetate (24.1332 g), CoCl₂ (15.5807 g), H₂O (51.884 mL) and ethanol (278.41 mL) was used. The H₂O:alkoxide ratio was greater than 4:1. The nominal catalyst composition was Cr_{0.2}Co_{0.2}(SiO₂₋₁(OH)₂₁)_{0.6}.

### EXAMPLE 25

An identical procedure as described in Example 20 was employed, except that the reagents and stoichiometries used were adjusted as indicated below. Tetraethylorthosilicate (118.75 g) was dissolved in ethanol (435.84 mL). An aqueous solution consisting of ruthenium chloride (6.2223), H₂O (41.07 mL) and ethanol (3435.84 mL) was used as well as HNO₃ (3.82 mL) and glacial acetic (4.89 mL). The H₂O:alkoxide ratio was 4:1. The nominal catalyst composition was Ru_{0.05}(SiO₂₋₁(OH)₂₁)_{0.95}.

### EXAMPLE 26

An identical procedure as described in Example 20 was employed, except that the reagents and stoichiometries used were adjusted as indicated below. Niobium ethoxide (151.11 g) was dissolved in ethanol (363.20 mL) ml of ethanol. An aqueous solution consisting of ruthenium chloride (5.1858 g) in H₂O (42.786 mL) and ethanol (363.20 mL) was used as well as HNO₃ (3.979 mL) and glacial acetic acid(5.096 mL). The H₂O:alkoxide ratio was 5:1. The nominal catalyst composition was Ru_{0.05}(NbO_{2.5-1}(OH)₂₁)_{0.95}.

### EXAMPLE 27

An identical procedure as described in Example 20 was employed, except that the reagents and stoichiometries used were adjusted as indicated below. Titanium n-butoxide (194.01 g) was dissolved in ethanol (435.84 mL). An aqueous solution consisting of ruthenium chloride (6.2223 g) in H₂O (41.08 mL) and ethanol (45.843 mL) was used as well as HNO₃ (3.82 mL) and glacial acetic acid(4.89 mL). The H₂O:alkoxide ratio was 4:1. The nominal catalyst composition was Ru_{0.05}(TiO₂₋₁(OH)₂₁)_{0.95}.

### EXAMPLE 28

An identical procedure as described in Example 20 was employed, except that the reagents and stoichiometries used were adjusted as indicated below. Zirconium n-propoxide (70 wt % in propanol (186.709 g) was dissolved in ethanol (435.84 mL). An aqueous solution consisting of ruthenium chloride (6.2229 g) in H₂O (41.075 mL) and ethanol (435.84 mL) was used as well as HNO₃ (3.82 mL) and glacial acetic acid (4.89 mL). The H₂O:alkoxide ratio was 4:1. The nominal catalyst composition was Ru_{0.05}(ZrO₂₋₁(OH)₂₁)_{0.95}.

### EXAMPLE 29

Commercially available Acrylonitrile Catalyst (catalyst 41/49) from Standard Oil Company of Ohio was used.

### EXAMPLE 30

A 1.75 M aqueous ammonium molybdate solution ((NH)₄)₆Mo₇O₂₄•4H₂O, 1.714 mL) was combined with 0.5 M bismuth nitrate solution ( prepared by dissolving Bi(NO₃)₃•9H₂O in 1 M nitric acid, 32.0 mL), 1.0 M aqueous nickel chloride solution (NiCl₂•6H₂O, 0.5 mL) and 1.0 M aqueous cobalt nitrate solution (Co(NO₃)₂•yH₂O, 0.5 mL) in a 150 mL petri dish with gentle swirling. The entire mixture was rapidly frozen with liquid nitrogen and evacuated to dryness under vacuum in a two section Virtis freeze drying unit (Virtis Freezemobile and Unitop drying unit) for at least 24 hours; the drier shelves were kept refrigerated to approximately -10°C during this evacuation. The final dried material was calcined in air to 400°C for 5 hours. The calcined powder was pelletized at 20,000 psig (138 mPa) and granulated on -40, +60 mesh (-0.42, +0.25 mm) screens prior to use.The nominal catalyst composition was Mo_{0.375}Bi_{0.5}Ni_{0.063}Co_{0.063}O₁.

**TABLE 1**

| Xerogels (Au, Cr) | | | | | | | |
|---|---|---|---|---|---|---|---|
| EX. | Cat. | Wt. g | T °C | CT s | %TMA Conv. | %DMF Sel. | %MMF Sel. |
| 1 | Au_{0.025}Cr_{0.025}Mg_{0.95} | 0.46 | 176 | 1.14 | 0.9 | 0.0 | 0.0 |
| | | | 226 | 1.02 | 2.7 | 26.2 | 0.0 |
| | | | 276 | 0.93 | 15.5 | 20.6 | 0.0 |
| 2 | Au_{0.01}Cr_{0.01}Al_{0.98} | 0.30 | 176 | 1.21 | 18.5 | 35.0 | 0.0 |
| | | | 226 | 1.09 | 54.7 | 24.3 | 3.9 |
| | | | 275 | 0.99 | 92.9 | 6.5 | 4.5 |
| 3 | Au_{0.025}Mg_{0.9025}Cr_{0.015}Si_{0.0475} | 0.59 | 176 | 0.84 | 2.0 | 0.0 | 0.0 |
| | | | 226 | 0.76 | 1.8 | 26.3 | 0.0 |
| | | | 276 | 0.69 | 22.8 | 6.7 | 24.8 |
| 4 | Cr_{0.025}Al_{0.975} | 0.28 | 175 | 0.87 | 4.9 | 0.0 | 0.0 |
| | | | 225 | 0.79 | 16.0 | 10.5 | 0.0 |
| | | | 274 | 0.71 | 73.4 | 2.4 | 1.3 |
| 5 | Au_{0.025}Al_{0.95}Cr_{0.025} | 0.40 | 176 | 1.14 | 50.3 | 44.6 | 8.4 |
| | | | 225 | 1.03 | 92.7 | 30.2 | 21.0 |
| | | | 274 | 0.94 | 98.3 | 0.7 | 5.5 |
| 6 | Au_{0.075}Cr_{0.025}Al_{0.9025}Mg_{0.0475} | 0.29 | 175 | 0.87 | 2.5 | 63.7 | 0.0 |
| | | | 225 | 0.78 | 17.7 | 40.7 | 6.0 |
| | | | 274 | 0.71 | 72.7 | 15.2 | 4.9 |
| 7 | Au_{0.025}Cr_{0.025}Al_{0.9025}Si_{0.0475} | 0.33 | 176 | 0.85 | 19.2 | 49.5 | 5.2 |
| | | | 226 | 0.76 | 75.5 | 25.6 | 8.8 |
| | | | 275 | 0.69 | 82.7 | 0.5 | 1.0 |
| 8 | Au_{0.025}Cr_{0.025}Mg_{0.32}Al_{0.32}Si_{0.32} | 0.55 | 175 | 1.11 | 3.3 | 0.0 | 0.0 |
| | | | 225 | 1.00 | 3.2 | 23.5 | 0.0 |
| | | | 275 | 0.90 | 7.3 | 20.3 | 0.0 |
| 9 | Au_{.025}Cr_{.025}Si_{.9025}Mg_{.0475} | 0.53 | 176 | 1.20 | 2.0 | 0.0 | 0.0 |
| | | | 226 | 1.08 | 9.6 | 11.5 | 0.0 |
| | | | 276 | 0.99 | 35.5 | 12.3 | 9.3 |

**TABLE 2**

| Oxometallates | | | | | | | |
|---|---|---|---|---|---|---|---|
| EX. | Cat. | Wt. g | T °C | CT s | %TMA Conv. | %DMF Sel. | %MMF Sel. |
| 10 | H₇P₂Mo₁₇VO₆₂ | 0.91 | 175 | 1.12 | 95.5 | 9.2 | 1.5 |
| | | | 224 | 1.02 | 99.4 | 18.7 | 15.6 |
| | | | 275 | 0.91 | 95.2 | 5.4 | 24.1 |
| 11 | HₚPMo₁₀CuVO₄₀ | 0.94 | 176 | 1.11 | 84.7 | 11.9 | 1.0 |
| | | | 225 | 1.00 | 86.1 | 15.8 | 10.6 |
| | | | 275 | 0.91 | 33.1 | 7.8 | 21.9 |
| 12 | Cu_{0.2}HₙPMo₁₀VCrO₄₀ | 0.81 | 176 | 1.10 | 31.6 | 15.9 | 0.0 |
| | | | 226 | 0.99 | 100 | 0.0 | 0.0 |
| | | | 275 | 0.90 | 100 | 0.0 | 0.0 |
| 13 | H₈PMo₁₀VZnO₄₀ | 0.87 | 176 | 1.10 | 69.9 | 9.6 | 0.9 |
| | | | 226 | 0.99 | 89.2 | 23.3 | 16.7 |
| | | | 275 | 0.90 | 73.0 | 7.5 | 14.9 |
| 14 | H₂Ag₂PMo₁₁VO₄₀ | 1.04 | 175 | 0.87 | 53.0 | 10.3 | 1.2 |
| | | | 224 | 0.80 | 99.5 | 15.1 | 18.5 |
| | | | 274 | 0.71 | 53.6 | 8.6 | 33.0 |
| 15 | H₃AgPMo₁₁VO₄₀ | 1.03 | 175 | 1.27 | 60.1 | 10.6 | 1.3 |
| | | | 224 | 1.14 | 99.3 | 14.8 | 0.0 |
| | | | 274 | 1.03 | 100 | 3.8 | 0.0 |
| 16 | HₘPMoV₂CuO₄₀ | 0.76 | 175 | 1.12 | 100 | 19.9 | 2.6 |
| | | | 225 | 1.01 | 99.5 | 18.8 | 22.2 |
| | | | 275 | 0.91 | 98.5 | 10.1 | 23.0 |
| 17 | H₈PMo₁₀VCoO₄₀ | 0.70 | 176 | 1.10 | 68.2 | 19.6 | 2.1 |
| | | | 226 | 0.99 | 100 | 26.3 | 25.3 |
| | | | 276 | 0.90 | 95.4 | 7.9 | 27.5 |
| 18 | Cs₄PMo₁₁VO₄ | 1.02 | 175 | 0.87 | 18.8 | 27.9 | 13.7 |
| | | | 224 | 0.78 | 55.8 | 16.5 | 15.3 |
| | | | 273 | 0.71 | 100 | 20.6 | 19.9 |

**TABLE 3**

| Xerogel Oxometallates Composites | | | | | | | |
|---|---|---|---|---|---|---|---|
| EX. | Cat. | Wt. g | T °C | CT s | %TMA Conv. | %DMF Sel. | %MMF Sel. |
| 19 | Ru_{0.05}(HPA)_{0.05}(TiO₂)_{0.9} | 0.35 | 176 | 1.10 | 7.4 | 37.5 | 0.0 |
| | | | 226 | 0.99 | 29.1 | 23.9 | 6.3 |
| | | | 276 | 0.90 | 100 | 12.8 | 0.0 |
| 20 | Ru_{0.05}(HPA)_{0.05}(ZrO₂)_{0.9} | 0.61 | 176 | 1.10 | 100 | 37.5 | 0.0 |
| | | | 226 | 0.99 | 99.8 | 23.9 | 0.0 |
| | | | 276 | 0.90 | 98.1 | 12.8 | 0.0 |
| 21 | Ru_{0.05}(HPA)_{0.05}(NbO_{2.5})_{0.9} | 0.28 | 175 | 1.11 | 3.9 | 14.4 | 0.0 |
| | | | 225 | 1.00 | 19.3 | 17.1 | 0.0 |
| | | | 274 | 0.91 | 100 | 5.9 | 0.0 |

**TABLE 4**

| Xerogels (Ti, Si, Ni,Zr) | | | | | | | |
|---|---|---|---|---|---|---|---|
| EX. | Cat. | Wt. g | T °C | CT s | %TMA Conv. | %DMF Sel. | %MMF Sel. |
| 22 | Cr_{0.2}(TiO₂₋₁(OH)₂₁)_{0.8} | 0.48 | 176 | 1.11 | 7.2 | 0.0 | 0.0 |
| | | | 226 | 1.00 | 18.2 | 9.1 | 5.4 |
| | | | 276 | 0.91 | 53.2 | 8.3 | 22.6 |
| 23 | Cr_{0.2}(SiO₂₋₁(OH)₂₁)_{0.8} | 0.43 | 175 | 1.10 | 1.1 | 0.0 | 0.0 |
| | | | 225 | 0.99 | 2.2 | 22.5 | 0.0 |
| | | | 273 | 0.90 | 54.2 | 17.5 | 22.4 |
| 24 | Cr_{0.2}Co_{0.2}(SiO₂₋₁(OH)₂₁)_{0.6} | 0.43 | 176 | 0.83 | 5.3 | 3.4 | 0.0 |
| | | | 226 | 0.74 | 12.5 | 3.5 | 0.0 |
| | | | 276 | 0.68 | 15.2 | 10.4 | 2.8 |
| 25 | Ru_{0.05}(SiO₂₋₁(OH)₂₁)_{0.95} | 0.51 | 176 | 1.10 | 1.9 | 0.0 | 44.5 |
| | | | 226 | 0.99 | 5.7 | 9.2 | 6.8 |
| | | | 275 | 0.90 | 9.4 | 10.5 | 15.0 |
| 26 | Ru_{0.05}(NbO_{2.5-1}(OH)₂₁)_{0.95} | 0.64 | 176 | 0.82 | 1.7 | 0.0 | 0.0 |
| | | | 226 | 0.74 | 3.2 | 11.1 | 10.8 |
| | | | 276 | 0.68 | 10.0 | 43.9 | 6.9 |
| 27 | Ru_{0.05}(TiO₂₋₁(OH)₂₁)_{0.95} | 0.55 | 175 | 0.86 | 3.2 | 34.2 | 0.0 |
| | | | 224 | 0.78 | 3.1 | 8.1 | 0.0 |
| | | | 274 | 0.71 | 7.6 | 23.3 | 0.0 |
| 28 | Ru_{0.05}(ZrO₂₋₁(OH)₂₁)_{0.95} | 0.60 | 175 | 1.10 | 8.8 | 13.5 | 10.4 |
| | | | 225 | 0.99 | 24.5 | 12.3 | 2.7 |
| | | | 274 | 0.90 | 57.5 | 3.9 | 1.9 |

**TABLE 5**

| Multicomponent Molybdate Catalysts | | | | | | | |
|---|---|---|---|---|---|---|---|
| EX. | Cat. | Wt. g | T °C | CT s | %TMA Conv. | %DMF Sel. | %MMF Sel. |
| 29 | ACRN CAT. 41/49 | 0.34 | 175 | 0.57 | 3.0 | 9.9 | 0.0 |
| | | | 225 | 0.51 | 9.0 | 19.7 | 0.0 |
| | | | 275 | 0.47 | 31.2 | 28.3 | 6.7 |
| 30 | Mo_{0.375}Bi_{0.5}Ni_{0.063}Co_{0.063}O₁ | 1.10 | 176 | 1.14 | 3.2 | 0.0 | 0.0 |
| | | | 226 | 1.03 | 8.2 | 11.8 | 0.0 |
| | | | 276 | 0.94 | 47.0 | 16.2 | 7.1 |

## Claims

1. A process for the preparation of a substituted formamide, said processing comprising:
(i) contacting oxygen in the vapor phase with a compound of the formula CH₃NR₂, wherein R is selected from the group consisting of C₁ to C₈ alkyl, phenyl, naphthyl and C₁ to C₄ alkyl substituted phenyl or mixtures thereof; in the presence of a catalyst, said catalyst selected from the group consisting of:
(a) xerogels or aerogels of the formula AₓAu_{y}M_{1-(x+y)}, wherein A is selected from the group consisting of Cu, Cr, Ru, Co and combinations thereof, M is selected from the group consisting of Al, Mg, Nb, Si, Ti, Zr and combinations thereof, x is from 0.001 to 0.5 and y is from 0 to 0.2;
(b) heteropolyacids selected from the group consisting of H₄PMo₁₁VO₄₀, H₅PMo₁₀V₂O₄₀, H₆PMo₉V₃O₄₀ and H₇P₂Mo₁₇VO₆₂;
(c) metal-substituted heteropolyacids selected from the group consisting of H_{4+(6-z)}PMo₁₀VEO₄₀, H_{5+(6-z)}PMo₉V₂EO₄₀, H_{6+(6-z)}PMo₈V₃EO₄₀ and H_{7+(6-z)}P₂Mo₁₆VEO₆₂, wherein E is an element selected from the group consisting of Cu, Cr, Zn and Co and z is the valence of E;
(d) heteropolyacid salts selected from the group consisting of Q_{q}H₄PMo₁₁VO₄₀, Q_{q}H₅PMo₁₀V₂O₄₀, Q_{q}H₆PMo₉V₃O₄₀, Q_{q}H₇P₂Mo₁₇VO₆₂, Q_{q}H_{4+(6-z)}PMo₁₀VEO₄₀, Q_{q}H_{5+(6-z)}PMo₉V₂EO₄₀, Q_{q}H_{6+(6-z)}PMo₈V₃EO₄₀ and Q_{q}H_{7+(6-z)}P₂Mo₁₆VEO₆₂ wherein Q is selected from the group consisting of Ag and Cs and q is from 0 to the number of hydrogen atoms multiplied by 0.5 when Q is Ag, and q is from 0 to the number of hydrogen atoms multiplied by 1 when Q is Cs; and
(e) metal oxides of the formula (BiₜCoᵤNiᵥ)_{w}Mo_{1-w}Oₐ, wherein t, u, and v each are independently from 0 to 1 and t+u+v = 1, w is from 0.01 to 0.75 and a is from 1.125 to 4.875; and
(ii) recovering said substituted formamide.

2. The process of Claim 1 wherein said substituted formamide is an N-substituted formamide having the formula HC(O)NR₂.

3. The process of Claim I wherein the oxygen is selected from the group consisting of substantially pure oxygen, air and oxygen mixed with inert gas.

4. The process of Claim 1 wherein said contacting is done at a temperature of from about 125° C to about 450° C and at pressure conditions from about 1 atm to about 10 atm.

5. The process of Claim 4 wherein said temperature is from about 150° C to about 275° C.

6. The process of any one of Claims 1 to 4 wherein the substituted formamide is recovered by fractional distillation.

7. The process of Claim 1 wherein the xerogel or aerogel of (a) is prepared from metal oxides selected from the group consisting of aluminum isopropoxide, titanium isopropoxide and zirconium isopropoxide.

8. The process of Claim 1 wherein the xerogel or aerogel of (a) is prepared from inorganic metal colloids selected from the group consisting of alumina sols, colloidal zirconia sols, colloidal titania sols, mixed oxide colloid sols, colloidal silica sols, ternary or higher order oxide sols, and mixtures of thereof.

9. The process of Claim 8 wherein the xerogel or aerogel of Claim 1 is prepared from preformed aquasols.

10. A composition of matter, comprising: xerogels or aerogels of the formula AₓAu_{y}M_{1-(x+y)} wherein A is selected from the group consisting of Cu, Cr, Ru, Co and combinations thereof, M is selected from the group consisting of Al, Mg, Nb, Si, Ti, Zr and combinations thereof, wherein x is from 0.001 to 0.5 and wherein y is from 0 to 0.2.
